# EUROPEAN PATENT APPLICATION

(11) **EP 1 837 022 A1**
(43) Date of publication of application: **26.09.2007**
(21) Application number: 05816773.5
(22) Date of filing: 13.12.2005
(51) Int. Cl.: A61K 31/485, A61P 1/08, A61P 25/02, C07D 489/00

(54) **INDOLOMORPHINAN DERIVATIVE HAVING CARBOXY IN 6'-POSITION**

(30) Priority: 14.12.2004 JP 2004360966; 04.02.2005 JP 2005028927; 08.04.2005 JP 2005111912; 11.10.2005 JP 2005296045; 11.10.2005 JP 2005296033
(71) Applicant: Shionogi Co., Ltd., Osaka-shi, Osaka 541-0045 (JP)
(72) Inventor: INAGAKI, Masanao, Shionogi & Co., Ltd., Osaka-shi, Osaka 553-0002 (JP); KUME, Masaharu, Shionogi & Co., Ltd., Osaka-shi, Osaka 553-0002 (JP)
(74) Representative: Kador & Partner
(86) International application number: PCT/JP2005/022820
(87) International publication number: WO 2006/064779

(57) **Abstract**

A novel compound useful as a therapeutic and/or preventive agent for nausca and/or vomiting. It is a compound represented by the formula (I): (wherein R¹ is hydrogen or -CHR^{A}R^{B} (wherein R^{A} is lower alkoxycarbonyloxy, etc. and R^{B} is hydrogen or methyl)), a pharmaceutically acceptable salt thereof, or a solvate of either.

## Description

### TECHNICAL FIELD

The present invention relates to indolomorphinan derivatives useful as remedies and/or preventives for nausea, retching and/or emesis, especially the retching and/or emesis induced by the compounds having opioid receptor (such as opioid µ receptor) agonism.

### BACKGROUND ART

Opioid µ receptor agonists such as morphine are used as analgesics very effective for cancer pain patients, but as side effects, they induce strong nausea, retching, emesis, anuresis, pruritus, etc. Various antiemetics are clinically used, but they do not exhibit a sufficient effect. Also for improving the QOL of patients, excellent side-effect relieving agents are demanded.
Patent documents 1 to 3 respectively describe to the effect that compounds similar to the compounds of the present invention are effective for curing or preventing the nausea and emesis induced by opioid µ agonists, but do not particularly describe the compounds of the present invention.
Patent document 1: WO, 2004/007503, A
Patent document 2: WO, 95/31463, A
Patent document 3: WO, 94/07896, A

### DISCLOSURE OF THE INVENTION

### PROBLEM TO BE SOLVED BY THE INVENTION

This invention provides indolomorphinan derivatives useful as remedies and/or preventives for the retching and/or emesis caused by opioid receptors.

### MEANS FOR SOLVING THE PROBLEM

This invention provides the following:
(1) A compound represented by the following formula (I): 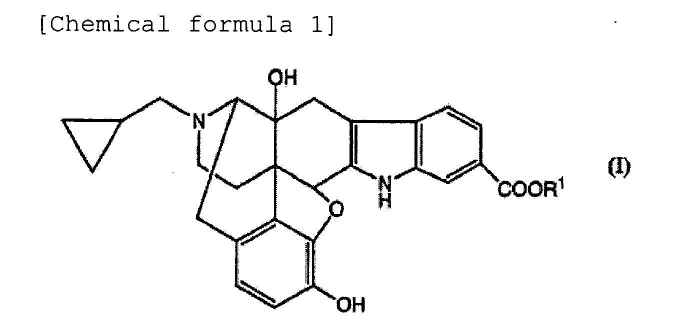 {where R¹ denotes hydrogen or -CHR^{A}R^{B} (where R^{A} denotes a lower alkoxycarbonyloxy, cycloalkoxycarbonyloxy, acyloxy or a group represented by 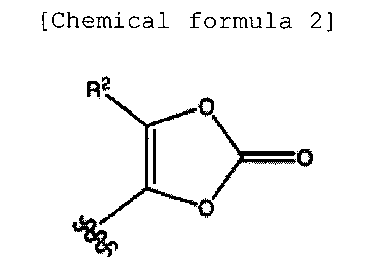 (where R² denotes hydrogen or a lower alkyl with or without a substituent), and R^{B} denotes hydrogen or methyl}, or any of pharmaceutically acceptable salts thereof or any of solvates thereof.
(2) A compound or any of pharmaceutically acceptable salts thereof or any of solvates thereof, according to said (1), wherein R¹ denotes hydrogen.
(3) A drug composition comprising the compound or any of pharmaceutically acceptable salts thereof or any of solvates thereof as set forth in said (1).
(4) An opioid receptor antagonist comprising the compound or any of pharmaceutically acceptable salts thereof or any of solvates thereof as set forth in said (1).
(5) A drug composition, according to said (3), wherein the compound or any of pharmaceutically acceptable salts thereof or any of solvates thereof as set forth in said (2) is a medicinal active ingredient.
(6) A drug composition, according to said (3), which is a remedy and/or preventive for retching and/or emesis.
(7) A drug composition, according to said (3), which is an agent for relieving and/or preventing the side effect induced by a compound having opioid receptor agonism.
(8) A remedy and/or preventive, according to said (7), wherein the side effect is retching and/or emesis.
(9) A remedy and/or preventive, according to said (7) or (8), wherein the compound having opioid receptor agonism is morphine, oxycodone, any of pharmaceutically acceptable salts thereof or any of solvates thereof.

(10) Use of the compound or any of pharmaceutically acceptable salts thereof or any of solvates thereof as set forth in said (1) or (2), for producing a drug having opioid receptor antagonism.
(11) Use of the compound or any of pharmaceutically acceptable salts thereof or any of solvates thereof as set forth in said (1) or (2), for producing a drug for curing and/or preventing retching and/or emesis.
(12) Use of the compound or any of pharmaceutically acceptable salts thereof or any of solvates thereof as set forth in said (1) or (2), for producing a drug for relieving and/or preventing the side effect induced by a compound having opioid receptor agonism.
(13) A method for inhibiting an opioid receptor, characterized by administering the compound or any of pharmaceutically acceptable salts thereof or any of solvates thereof as set forth in said (1) or (2).
(14) A method for curing and/or preventing retching and/or emesis, characterized by administering the compound or any of pharmaceutically acceptable salts thereof or any of solvates thereof as set forth in said (1) or (2).
(15) A method for relieving and/or preventing the side effect induced by a compound having opioid receptor agonism, characterized by administering the compound or any of pharmaceutically acceptable salts thereof or any of solvates thereof as set forth in said (1) or (2).
(16) An analgesic comprising a compound having opioid receptor agonism and the compound or any of pharmaceutically acceptable salts thereof or any of solvates thereof as set forth in said (1) or (2) in an amount effective for relieving and/or preventing the side effect induced by the administration of said compound, in combination.
(17) An analgesic comprising a compound having opioid receptor agonism and the compound or any of pharmaceutically acceptable salts thereof or any of solvates thereof as set forth in said (1) or (2) in an amount effective for curing and/or preventing the retching and/or emesis induced by the administration of said compound, in combination.

### EFFECT OF THE INVENTION

The compounds (I) of this invention respectively have an action of curing and/or preventing retching and/or emesis, especially the retching and/or emesis induced by a compound having opioid receptor (for example, opioid µ receptor) agonism and is useful as an agent for relieving the side effect of patients who will be or is being administered with a compound having opioid receptor agonism.

### THE BEST MODES FOR CARRYING OUT THE INVENTION

In this specification, the term "halogen" includes fluorine, chlorine, bromine and iodine.
The term "lower alkyl" is a straight chain or branched alkyl with 1 to 10 carbon atoms, preferably 1 to 6 carbon atoms, more preferably 1 to 3 carbon atoms, and includes, for example, methyl, ethyl, n-propyl, isopropyl, n-butyl, isobutyl, sec-butyl, tert-butyl, n-pentyl, isopentyl, neopentyl, hexyl, isohexyl, n-heptyl, isoheptyl, n-octyl, isooctyl, n-nonyl, n-decyl, etc. Preferred are methyl and ethyl.
The substituent of the term "lower alkyl with or without a substituent" includes a halogen, lower alkoxy, acyl or acyloxy, etc.
The lower alkyl portions of the terms "lower alkoxy, " "lower alkoxycarbonyloxy," "aryl lower alkyl," "tri- lower alkylsilyl," "lower alkyldiarylsilyl," "triaryl lower alkylsilyl," "lower alkoxy lower alkyl," "lower alkoxy lower alkoxy lower alkyl, " "lower alkylthio lower alkyl," "aryl lower alkyloxy lower alkyl" and "lower alkylsulfonyl" are the same as said "lower alkyl."

The cycloalkane portion of the term "cycloalkoxycarbonyloxy" is an alicyclic carbon ring with 3 to 8 carbon atoms, and particularly includes cyclopropane, cyclobutane, cyclopentane, cyclohexane, cycloheptane and cyclooctane.
The term "aryl" includes phenyl, naphthyl, anthryl, phenanthryl, etc., and especially preferred is phenyl.
The aryl portions of the terms "arylcarbonyl," "aryl lower alkyl", "lower alkyldiarylsilyl," "triaryl lower alkylsilyl," "aryl lower alkyloxy lower alkyl" and "arylsulfonyl" are the same as said "aryl."
The term "acyl" includes (1) a straight chain or branched alkylcarbonyl or alkenylcarbonyl respectively with 1 to 10 carbon atoms, preferably 1 to 6 carbon atoms, most preferably 1 to 4 carbon atoms, (2) a cycloalkylcarbonyl with 4 to 9 carbon atoms, preferably 4 to 7 carbon atoms and (3) an arylcarbonyl with 7 to 11 carbon atoms.
In this case, the alkyl portion of the term "alkylcarbonyl" is the same as said "lower alkyl."
The alkenyl portion of the term "alkenylcarbonyl" is a straight chain or branched alkenyl having one or more double bonds at given positions and with 2 to 10 carbon atoms, preferably 2 to 8 carbon atoms, more preferably 3 to 6 carbon atoms, and particularly includes vinyl, allyl, propenyl, isopropenyl, butenyl, isobutenyl, prenyl, butadienyl, pentenyl, isopentenyl, pentadienyl, hexenyl, isohexenyl, hexadienyl, heptenyl, octenyl, nonenyl, decenyl, etc.
The cycloalkyl portion of the term "cycloalkylcarbonyl" is a carbocyclic group respectively with 3 to 10 carbon atoms, preferably 3 to 8 carbon atoms, more preferably 4 to 8 carbon atoms, and includes, for example, cyclopropyl, cyclobutyl, cyclopentyl, cyclohexyl, cycloheptyl, cyclooctyl, cyclononyl and cyclodecyl, etc.
Particular examples of the term "acyl" include formyl, acetyl, propionyl, butyryl, isobutyryl, valeryl, pivaloyl, hexanoyl, acryloyl, propioloyl, methacryloyl, crotonoyl, cyclopropylcarbonyl, cyclohexylcarbonyl, cyclooctylcarbonyl, benzoyl, etc.
The acyl portion of the term "acyloxy" is the same as said "acyl."

In this specification, the term "solvate" includes, for example, a solvate with an organic solvent, hydrate, etc. When a hydrate is formed, it may be coordinated with a given number of water molecules.
The compounds (I) include pharmaceutically acceptable salts. As examples of them, enumerated are salts with alkali metals (lithium, sodium, potassium, etc.), alkaline earth metals (magnesium, calcium, etc.), salts with ammonium, organic bases and amino acids, and salts with inorganic acids (hydrochloric acid, sulfuric acid, nitric acid, hydrobromic acid, phosphoric acid, hydroiodic acid, etc.) and organic acids (acetic acid, citric acid, lactic acid, tartaric acid, oxalic acid, maleic acid, fumaric acid, mandelic acid, glutaric acid, malic acid, benzoic acid, phthalic acid, benzenesulfonic acid, p-toluenesulfonic acid, methanesulfonic acid, ethanesulfonic acid, etc.). Especially hydrochloric acid, phosphoric acid, tartaric acid, methanesulfonic acid, etc. are preferred. These salts can be formed by usually used methods.
Further, the compounds (I) are not limited to any specific isomer, and include all possible isomers and racemic modifications.

Any of the compounds (I) of this invention can be produced by the following method. 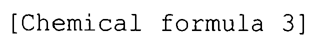 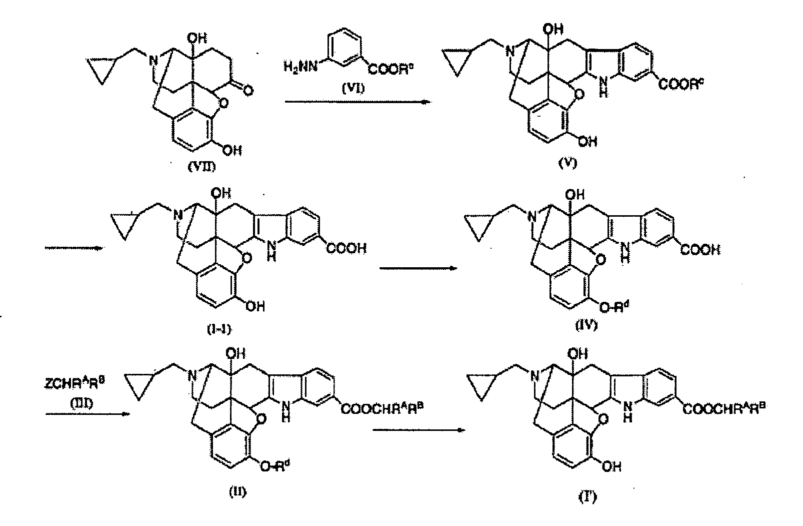 (where R^{c} denotes hydrogen or lower alkyl; R^{d} denotes a hydroxy protective group; Z denotes hydroxy or halogen; and the other symbols are as defined before.)
At first, publicly known naltrexone (VII) and a m-hydrazinobenzoic acid lower alkyl ester (VI) are made to react with each other in an appropriate solvent (for example, methanol, ethanol, isopropanol, dimethylformamide, dimethyl sulfoxide, acetic acid, propionic acid or any of mixtures thereof, etc.) in the presence of an acid (for example, hydrochloric acid, sulfuric acid, phosphoric acid, methanesulfonic acid, p-toluenesulfonic acid, acetic acid, polyphosphoric acid, etc.) at about 0°C to about 200°C, preferably about 20°C to about 150°C for about 5 minutes to about 24 hours, preferably for about 1 hour to about 5 hours, to obtain a compound (V).
In the case where R^{c} of the obtained compound (V) is a lower alkyl, hydrolysis is performed to obtain a compound (I-1). The hydrolysis reaction can be performed in an appropriate solvent (dioxane, tetrahydrofuran, methanol, ethanol, water or any of mixtures thereof, etc.) in the presence of a base (sodium hydroxide, potassium hydroxide, lithium hydroxide, barium hydroxide, potassium carbonate, sodium carbonate, etc.) at about 0°C to about 200°C, preferably about 30°C to about 100°C for about 5 minutes to about 24 hours, preferably about 1 hour to about 5 hours.
Then, a hydroxy group of the obtained compound (I-1) is protected to obtain a compound (IV).
The hydroxy protective group is not especially limited, and as examples of it, enumerated are lower alkyls (methyl, tert-butyl, etc.), aryl lower alkyls (triphenylmethyl, benzyl, etc.), tri-lower alkylsilyls (trimethylsilyl, tert-butyldimethylsilyl, triethylsilyl, triisopropylsilyl, etc.), lower alkyldiarylsilyls (tert-butyldiphenylsilyl, etc.), triaryl lower alkylsilyls (tribenzylsilyl, etc.), lower alkoxy lower alkyls (methoxymethyl, 1-ethoxyethyl, 1-methyl-1-methoxyethyl, etc.), lower alkoxy lower alkoxy lower alkyls (methoxyethoxymethyl, etc.), lower alkylthio lower alkyls (methylthiomethyl, etc.), tetrahydropyranyls substituted or not substituted by a lower alkyl or lower alkoxy, etc. (tetrahydropyran-2-yl, 4-methoxytetrahydropyran-4-yl, etc.), tetrahydrothiopyranyls (tetrahydrothiopyran-2-yl, etc.), tetrahydrofuranyls (tetrahydrofuran-2-yl, etc.), tetrahydrothiofuranyls (tetrahydrothiofuran-2-yl, etc.), aryl lower alkyloxy lower alkyls (benzyloxymethyl, etc.), lower alkylsulfonyls (methanesulfonyl, ethanesulfonyl, etc.), acyls (acetyl, etc.), and arylsulfonyls substituted or non-substituted by a lower alkyl or lower alkoxy, etc. (p-toluenesulfonyl, etc.).
The reaction for introducing a protective group can be performed according to a conventional method. For example, in the case where tert-butyldimethylsilyl is used as the protective group, dimethylformamide, acetonitrile, tetrahydrofuran, dimethylformamide, dichloromethane, chloroform, toluene or any of mixtures thereof, etc. is used as a solvent, and a reaction with tert-butyldimethylsilyl chloride or t-butyldimethylsilyl trifluoromethanesulfonate, etc. is performed in the presence of imidazole, triethylamine or 2,6-lutidine, etc. The reaction can be performed at about -80°C to about 100°C, preferably about -20°C to about 25°C for about 5 minutes to about 24 hours, preferably about 1 hour to about 10 hours.

Into the obtained compound (IV), a CHR^{A}R^{B} group capable of being hydrolyzed in vivo is introduced.
In this reaction, for example, the compound (IV) and a halogenated alkyl (III) are made to react with each other in an appropriate solvent (dimethylformamide, dimethyl sulfoxide, acetonitrile, tetrahydrofuran, dichloromethane, diethyl ether, acetone, etc.) in the presence or absence of a base (an organic base such as pyridine, triethylamine or diisopropylethylamine, or an inorganic base such as sodium hydroxide, potassium t-butoxide, potassium carbonate, potassium hydrogencarbonate or sodium hydrogencarbonate) at about -80°C to about 100°C, preferably -20°C to about 60°C for about 5 minutes to about 24 hours, preferably about 30 minutes to about 3 hours, to obtain the intended compound.
Further, the compound (IV) and the compound (III) can also be dehydrated and condensed in the presence of an acid (hydrochloric acid, sulfuric acid or methanesulfonic acid) for esterification.

Finally the hydroxy protective group of the compound (II) is removed to obtain the compound (I').
The reaction for removing the protective group can be performed by a publicly known method suitable for the protective group. For example, in the case where tert-butyldimethylsilyl is used as the protective group, tetrahydrofuran, methanol, ethanol, acetic acid or water, etc. is used as the solvent, and fluoride anions (tetrabutylammonium fluoride, hydrofluoric acid, hydrofluoric acid-pyridine or potassium fluoride, etc.) are used for treatment, or an organic acid such as acetic acid, methanesulfonic acid, p-toluenesulfonic acid, trifluoroacetic acid or trifluoromethanesulfonic acid or an inorganic acid such as hydrochloric acid is used for treatment. The reaction can be performed at about -80°C to about 100°C, preferably about 0°C to about 40°C for about 5 minutes to about 24 hours, preferably about 30 minutes to about 10 hours, to obtain the intended compound (I').

The compounds of this invention are effective for curing and/or preventing the retching and emesis caused by acute indigestion, acute alcoholism, food poisoning, cold, gastric ulcer, duodenal ulcer, gastric cancer, ileus, appendicitis, peritonitis, cholecystitis, hepatitis, hepatic inflammation, cerebritis, meningitis, brain hypertension, head injury, motion sickness, vomiting of pregnancy, side effect of chemotherapy, side effect of radiotherapy, side effect of anti-cancer drug, etc., low digestive tract passage rates caused by pressure or stricture of digestive tract or by post-surgery intestinal adhesion, etc., brain pressure increase due to radiation irradiation to brain tumor, cerebral hemorrhage, meningitis and brain, etc. The compounds are especially effective for the nausea, retching and/or emesis induced by ingestion of a compound having opioid receptor (for example, opioid µ receptor) agonism.
The term "compound having opioid receptor agonism" particularly includes morphine, oxycodone, fentanyl, methadone, codeine, dihydrocodeine, hydromorphone, levorphanol, meperidine, propoxyphene, dextropropoxyphene, tramadol, pharmaceutically acceptable salts thereof, and solvates thereof. Especially in the case of morphine, oxycodone, pharmaceutically acceptable salts thereof and solvates thereof, the compounds of this invention are especially effective.

The compounds of this invention show strong opioid δ receptor antagonism. Further, as can be seen from the test examples described later, since the compounds of this invention are low in the ability to penetrate to the brain, the compounds little inhibit the analgesic action of the compounds having opioid receptor agonism administered to the patients of pain diseases {for example, cancerous pain (bone metastasis, neurothlipsis, increased intracranial pressure, soft tissue infiltration, muscle contraction pain, pain around viscera, muscles, fascias, loins and shoulder joints, post-surgery chronic pain), AIDS, etc.}, and show a high relieving effect against the side effect induced by receptor agonists. Further, the compounds have such features as high receptor selectivity, high oral absorbability, low toxicity, high stability in human plasma and high bioavailability and are very effective as drugs.

In the case where any of the compounds of this invention is administered for relieving the side effect induced by a compound having opioid receptor agonism, the compound of this invention can be taken pre, post, or simultaneous administration with the compound having opioid receptor agonism.
The administration interval between these two types of compounds is not especially limited. For example, in the case where the compound of this invention is administered after the compound having opioid receptor agonism has been administered, if the compound of this invention is administered in a period from immediately after the administration of the opioid receptor agonist till about 3 days later, preferably in a period from immediately after the administration till about one day later, it can act more effectively. Further, in the case where the compound of this invention is administered before the administration of the opioid receptor agonist, if the compound of this invention is administered in a period from about one day before the administration of the opioid receptor agonist till immediately before the administration, preferably in a period from about 12 hours before the administration till immediately before the administration, it can act more effectively.
In the case where the compound of this invention is administered as a remedy and/or preventive for retching and/or emesis, it can also be used together with another remedy and/or preventive for retching and/or emesis. For example, it can be used together with ondansetron hydrochloride, adrenocortical steroid (methylprednisolone, prednisolone, dexamethasone, etc.), prochlorperazine, haloperidol, timiperone, perphenazine, metoclopramide, domperidone, scopolamine, chlorpromazine hydrochloride or droperidol.
Further, the compound of this invention can also be administered as a mixture consisting of the compound of this invention and a compound having opioid receptor agonism or as a mixture consisting of the compound of this invention and another remedy and/or preventive for retching and/or emesis.
In the case where the compound of this invention is administered to the human, it can be administered orally as a powder, granules, tablets, capsules, pills, solution, etc. or parenterally as an injection, suppository, percutaneous absorption drug, inhalant, etc. Further, a medicinal additive such as an excipient suitable for the formulation of the compound, binder, wetting agent, disintegrator or lubricant can be mixed, as required, with the effective dose of the compound, to make a medicinal preparation.
The compound of this invention can also be mixed with a compound having opioid receptor agonism and/or another remedy and/or preventive for retching and/or emesis, and as required, various medicinal additives, to be provided as a mixture.
The dose depends on the disease condition, administration route, and the age or weight of the patient. In the case of oral administration to an adult, the dose is usually 1 µg to 10 g/day, preferably 0.01 to 200 mg/day, and in the case of parenteral administration, the dose is usually 0.1 µg to 1 g/day, preferably 0.1 to 2 g/day.

This invention is described below in more detail in reference to examples and test examples, but is not limited thereto or thereby.

### Example 1

### 17-cyclopropylmethyl-6,7-didehydro-4,5α-epoxy-3,14-dihydroxy-6 '-carboxy-6,7-2',3'-indolomorphinan

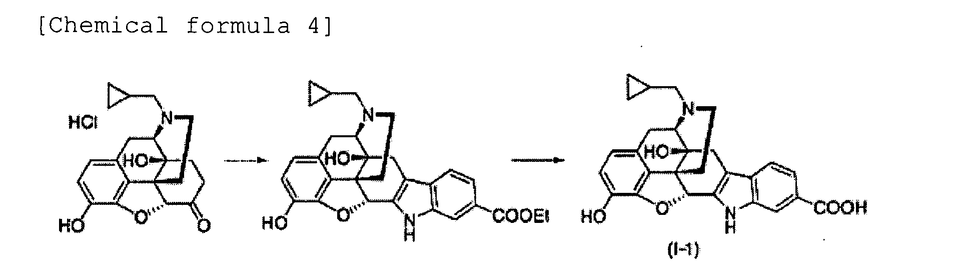

### (First process)

### 17-cyclopropylmethyl-6,7-didehydro-4,5α-epoxy-3,14β-dihydroxy-6 '-ethoxycarbonyl-6,7-2',3'-indolomorphinan

Publicly known naltrexone hydrochloride (500 mg, 1.32 mmol) and m-hydrazinobenzoic acid (221 mg, 1. 46 mmol) were suspended into 3 ml of ethanol, and the suspension was heated to 50°C and stirred. To the mixture, an ethanol (2 mL) solution of methanesulfonic acid (0.86 mL, 13.2 mmol) was added dropwise slowly, taking 10 minutes. After completion of dropwise addition, the mixture was stirred for 2 hours under reflux. The reaction solution was cooled to room temperature, and saturated sodium bicarbonate aqueous solution and ethyl acetate were added to the reaction solution. The organic layer was separated and washed with water and brine in this order. The organic layer was dried over sodium sulfate, and the solvent was removed. The residue was purified by silica gel column chromatography (chloroform:methanol = 99:1), to obtain 37 9 mg (59%) of the subject compound as a light yellow solid.
NMR(300MHz, CDCl₃)
δ 0.14-0.18(m, 2H), 0.55-0.59(m, 2H), 0.89(m, 1H), 1.41(t, 3H, J = 6.9 Hz), 1.75(d, 1H, J = 11.4 Hz), 2.20-2.91(m, 8H), 3.10(d, 1H, J = 18.6 Hz), 3.38(d, 1H, J = 6.3 Hz), 4.38(q, 2H, J = 6.9 Hz), 5.5(br s, 1H), 5.69(s, 1H), 6.46(d, 1H, J = 8.1 Hz), 6.55(d, 1H, J = 8.1Hz), 7.34(d, J = 8.4 Hz), 7.67(d, J = 8.4 Hz), 7.92(s, 1H), 8.36(s, 1H).

### (Second process)

### 17-cyclopropylmethyl-6,7-didehydro-4,5α-epoxy-3,14β-dihydroxy-6 '-carboxy-6,7-2',3'-indolomorphinan

To a methanol (2.4 mL) solution of the compound obtained in the first process (654 mg, 1.20 mmol), added was 2 mol/L-sodium hydroxide aqueous solution (2.4 mL), and the mixture was stirred for 1 hour under reflux. The reaction solution was cooled to room temperature and diluted by methanol, and the pH was adjusted to 6.0 using diluted hydrochloride acid. The precipitated crystals were collected by filtration, washed with water and dried, to obtain 534 mg (97%) of the subject compound as colorless crystals.
NMR(300MHz, d6-DMSO)
δ 0.14-0.18(m, 2H), 0.48-0.54(m, 2H), 0.90(m, 1H), 1.59(d, 1H, J = 11.7 Hz), 2.09-2.82(m, 8H), 3.07(d, 1H, J = 18.6 Hz), 5.55(s, 1H) , 6.49(d, 1H, J = 7.8 Hz), 6.52(d, 1H, J = 7.8Hz), 7.42(d, J = 8.4 Hz), 7.55(dd, J = 1.5, 8.4 Hz), 7.97(d, J = 1.5 Hz, 1H), 8.98(br s, 1H) , 11.54(s, 1H) .

### Example 2

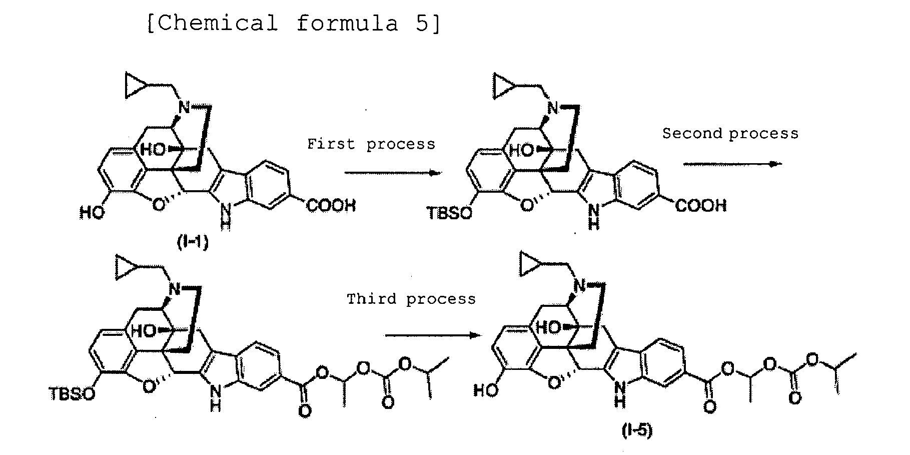

(where TBS denotes tert-butyldimethylsilyl, and the other symbols are as defined before.)

### (First process)

### 3-tert-butyldimethylsiloxy-17-cyclopropylmethyl-6,7-didehydro-4,5α-epoxy-14β-hydroxy-6'-carboxy-6,7-2',3'-indolomorphinan

To a dimethylformamide (5 mL) solution of the compound (I-1) obtained in Example 1 (459 mg, 1 mmol), added were tert-butyldimethylsilyl chloride (332 mg, 2.2 mmol) and imidazole (170 mg, 2.5 mmol), and the mixture was stirred overnight at room temperature. The reaction solution was poured into ice water-saturated sodium hydrogencarbonate aqueous solution, and the mixture was subjected to extraction with ethyl acetate. The organic layer was washed with saturated sodium chloride aqueous solution, dried over anhydrous sodium sulfate, and concentrated under reduced pressure. The obtained oily 3-tert-butyldimethylsiloxy-17-cyclopropylmethyl-6,7-didehydro-4,5α-epoxy-14β-hydroxy-6'-tert-butyldimethylsiloxycarbonyl-6,7-2',3'-indolomorphinan was used in the next reaction without being purified. To a mixed solution consisting of tetrahydrofuran (2 mL) and water (2 mL) of said oily substance, added was acetic acid (6 mL) , and the mixture was stirred at room temperature for 1.5 hours. The reaction solution was poured into ice water-2M sodium carbonate aqueous solution, for adjusting to pH = 6 - 7, and the mixture was subjected to extraction with ethyl acetate. The organic layer was washed with saturated sodium chloride aqueous solution, dried over anhydrous sodium sulfate, and concentrated under reduced pressure. The residue was purified by silica gel chromatography (chloroform/methanol = 10/1 → 8/1 → 6/1), to obtain the subject compound (405 mg, yield 71%) as colorless crystals.
¹H NMR (DMSO-d₆, δ ppm) : 0.07(s, 3H), 0.02(s, 3H), 0.11-0.21 (m, 2H), 0.45-0.57(m, 2H), 0.84(s, 9H), 0.88(m, 1H), 1.58(d, J = 11.4 Hz, 1H), 2.03-2.88(m, 9H), 3.10(d, J = 18.6 Hz, 1H), 4.75(br s, 1H), 5.60(s, 1H), 6.52(d, J = 8.1 Hz, 1H), 6.56(d, J = 8.1 Hz, 1H), 7.41(d, J = 8.4 Hz, 1H), 7.53(d, J = 8.4 Hz, 1H), 7.96(d, J = 0.6 Hz, 1H), 11.63(br s, 1H)

### (Second process)

### 3-tert-butyldimethylsiloxy-17-cyclopropylmethyl-6,7-didehydro-4,5α-epoxy-14β-hydroxy-6'-1"-(isopropoxycarbonyloxy)ethoxycarbo nyl-6,7-2',3'-indolomorphinan

To a dimethylformamide (2 mL) solution of the compound obtained in the first process (120 mg, 0.210 mmol), added were potassium carbonate (44 mg, 0.325 mmol) and 1-(isopropoxycarbonyloxy)ethyliodide (108 mg, 0.42 mmol) with ice cooling, and the mixture was stirred at the same temperature for 1 hour. The reaction solution was poured into ice water, and the mixture was subjected to extraction with ethyl acetate. The organic layer was washed with saturated sodium chloride aqueous solution, dried over anhydrous sodium sulfate, and concentrated under reduced pressure. The residue was purified by silica gel chromatography (chloroform/methanol = 100/1 → 50/1 → 30/1 → 20/1), to obtain the subject compound (118 mg, yield 80%) as a light orange foam.
¹H NMR(CDCl₃, δ ppm) : 0.02(d,J = 4.5 Hz, 3H), 0.02(d, J = 3.0 Hz, 3H), 0.13-0.25(m, 2H), 0.53-0.65(m, 2H), 0.88(d, J = 2.7 Hz, 9H), 0.95(m, 1H), 1.29(d, J = 6.3 Hz, 6H), 1.66(d, J = 5.1 Hz, 3H) , 1.79(d, J = 12.6 Hz, 1H), 2.22-2.97(m, 8H) , 3.15(d, J = 18.9 Hz, 1H), 3.39(br s, 1H), 4.90(m, 1H), 5.62(s, 1H), 6.54(dd, J = 2.1, 8.1 Hz, 1H), 6.58(dd, J= 2.1, 8.1 Hz, 1H) , 7.07(m, 1H) , 7.37(t, J = 8.1Hz, 1H), 7.68(m, 1H), 8.02(s, 1H), 8.36(d, J = 4.8 Hz, 1H)

### (Third process)

### 17-cyclopropylmethyl-6,7-didehyro-4,5α-epoxy-14β-hydroxy-6'-1"-(isopropoxycarbonyloxy)ethoxycarbonyl-6,7-2',3'-indolomorphina n

To a tetrahydrofuran (1.5 mL) solution of 3-tert-butyldimethylsiloxy-17-cyclopropylmethyl-6, 7-didehydro-4,5α-epoxy-14β-hydroxy-6'-11"-(isopropoxycarbonyloxy)ethoxycarb onyl-6, 7-2' , 3' -indolomorphinan (118 mg, 0.168 mmol) , added was 1M tetrabutylammonium fluoride/tetrahydrofuran solution (252 µL, 0.252 mmol), and the mixture was stirred at room temperature for 1 hour. The reaction solution was poured into ice water-saturated sodium hydrogencarbonate aqueous solution, and the mixture was subjected to extraction with ethyl acetate. The organic layer was washed with saturated sodium chloride aqueous solution, dried over anhydrous sodium sulfate, and concentrated under reduced pressure. The residue was purified by silica gel chromatography (chloroform/methanol = 100/1 → 50/1 → 30/1 → 20/1), and hexane/ethyl acetate was used for crystallization, to obtain the subject compound (67 mg, yield 68%) as colorless crystals.
¹H NMR(CDCl₃, δ ppm) : 0.10-0.22 (m, 2H), 0.50-0.64 (m, 2H), 0.88 (m, 1H), 1.30(d, J = 6.3 Hz, 6H), 1.67(d, J = 5.4 Hz, 3H), 1.76(d, J = 11.4 Hz, 1H), 2.20-2.92(m, 8H), 3.13(d, J = 19.2 Hz, 1H), 3.37 (d, J = 5.1 Hz, 1H) , 4.91(m, 1H) , 5.30(br s, 1H) , 5.67(s, 1H), 6.53 (d, J = 8.1 Hz, 1H), 6.61(d, J = 8.1 Hz, 1H), 7.05(m, 1H), 7.24 (m, 1H), 7.59(m, 1H), 7.84(br s, 1H), 8.43(br s, 1H)

Similarly other compounds (I) were synthesized. The structural formulae and physical constants thereof are shown below.

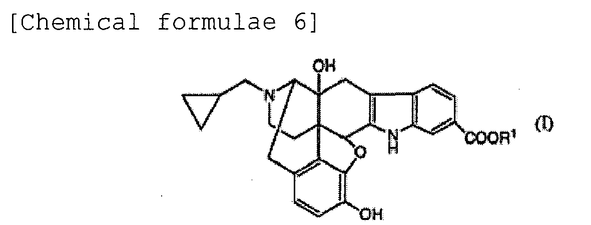

| | R1 | | R1 |
|---|---|---|---|
| I-1 | -H | I-6 | 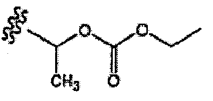 |
| I-2 | 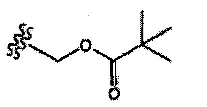 | I-7 | 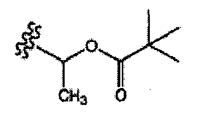 |
| I-3 | 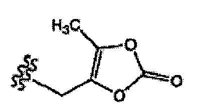 | I-8 | 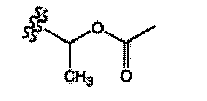 |
| I-4 | 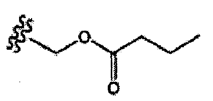 | I-9 | 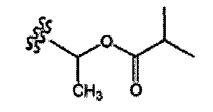 |
| I-5 | 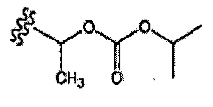 | I-10 | 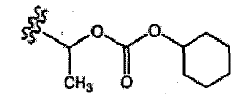 |

I-2
   ¹H NMR(CDCl₃, δppm): 0.15-0.2(m, 2H), 0.55-0.6(m, 2H), 0.85-0.95 (m, 1H), 1.21(s, 9H), 1.81(d, J = 13.5 Hz, 1H), 2.2-2.95(m, 8H), 3.16(d, J = 18.3 Hz, 1H), 3.35-3.45(m, 1H) 4.9-5.1(br s, 1H), 5.70 (s, 1H), 5.99-6.03(ABq, J = 5.4 Hz, 2H), 6.57(d, J = 8.3 Hz, 1H), 6.64(d, J = 8.3 Hz, 1H), 7.41(d, J = 8.6 Hz, 1H), 7.73(d, J = 8.6 Hz, 1H), 8.07(s, 1H), 8.47(br s, 1H).
I-3
   ¹H NMR(d₆-DMSO, δ ppm) : 0.1-0. 2 (m, 2H), 0.45-0.6(m, 2H), 0.85-0.95 (m, 1H), 1.60(d, J = 12.2 Hz, 1H), 2.1-2.85(m, 11H), 3.07(d, J = 18.7 Hz, 1H), 3.25-3.35(m, 1H), 4.78(br s, 1H), 5.20(s, 2H), 5.57 (s, 1H), 6.51(s, 2H), 7.48(d, J=8.3Hz, 1H), 7.59(d, J=8.3Hz, 1H), 8.01(s, 1H), 8.97(s, 1H), 11.62(s, 1H).
I-4
   ¹H NMR(d₆-DMSO, δ ppm): 0.1-0.2(m, 2H), 0.45-0.6(m, 2H), 0.8-1.0 (m, 4H), 1.5-1.65(m, 3H), 2.1-2.85(m, 10H), 3.07(d, J = 17.6 Hz, 1H), 3.25-3.4(m, 1H), 4.78(br s, 1H), 5.57(s, 1H), 5.95(s, 2H), 6.51(s, 2H), 7.49(d, J= 8.0 Hz, 1H), 7.55(d, J= 8.0 Hz, 1H), 8.01 (s, 1H), 8.97(s, 1H), 11.67(s, 1H).
I-6
   ¹H NMR(CDCl₃, δ ppm): 0.15-0.2(m, 2H), 0.55-0.6(m, 2H), 0.85-0.95 (m, 1H), 1.25-1.35(m, 3H), 1.67(d, J = 5.4 Hz, 3H), 1.75(d, J = 10.5 Hz, 1H), 2.2-2.9(m, 8H), 3.13(d, J = 18.6 Hz, 1H), 3.35-3.45 (m, 1H), 4.23(q, J = 7.0 Hz, 2H), 5. 1-5. 3(br s, 1H), 5.68(s, 1H), 6.52(d, J = 8.1 Hz, 1H), 6.60(d, J = 8.1 Hz, 1H), 7.06(q, J = 5.3 Hz, 1H), 7.22, 7.23(two d, J = 8.3 Hz, 1H), 7.56, 7.60(two d, J = 8.4 Hz, 1H), 7.82(s, 1H), 8.43(br s, 1H).
I-7
   ¹H NMR (d₆-DMSO, δ ppm) : 0.1-0.2(m, 2H), 0.45-0.6 (m, 2H), 0.85-0.95 (m, 1H), 1.15(s, 9H) , 1.57(d, J = 5.4 Hz), 1.55-1. 65 (m, 1H), 2.1-2.85 (m, 8H), 3.08(d, J = 18.0 Hz, 1H), 3.25-3.35(m, 1H), 4.77(br s, 1H), 5.57(s, 1H), 6.51(s, 2H), 6.96(q, J = 5.2 Hz, 1H), 7.47(d, J = 8.3 Hz, 1H), 7.56(d, J = 8.3 Hz, 1H), 7.99(s, 1H), 8.98 (s, 1H), 11.65(s, 1H).
I-8
   ¹H NMR(CDCl₃, δ ppm): 0.15-0.2(m, 2H), 0.55-0.6(m, 2H), 0.85-0.95 (m, 1H), 1.63(d, J = 5.4 Hz, 3H), 1.77(d, J = 11.4 Hz, 1H), 2.10, 2.11(two s, 3H), 2.2-2.9(m, 8H), 3.13(d, J = 18.6 Hz, 1H), 3.35-3.45 (m, 1H), 5.0-5.3(br s, 1H), 5.68(s, 1 H), 6.53(d, J = 8.1 Hz, 1H), 6.60(d, J = 8.1 Hz, 1H), 7.1-7.2(m, 1H), 7.31(d, J = 9.0 Hz, 1H), 7.62, 7.65(two d, J = 8.0 Hz, 1H), 7.93(s, 1H), 8.47(br s, 1H).
I-9
   ¹H NMR(CDCl₃, δ ppm) : 0.15-0.2(m, 2H), 0.55-0.6(m, 2H), 0.85-0.95 (m, 1H), 1.15-1.25(m, 6H), 1.62(d, J = 5.4 Hz, 3H), 1.78(d, J = 11.7 Hz, 1H), 2.2-2.9(m, 9H), 3.14(d, J = 18.3 Hz, 1H), 3.35-3.4 (m, 1H), 5.0-5.2(br s, 1H), 5.67(s, 1H), 6.5-6.6(m, 1H), 6.62(d, J = 7.8 Hz, 1H) , 7. 1-7.2 (m, 1H) , 7. 25, 7. 31 (two d, J = 7.8 Hz, 1H), 7.56, 7.51(two d, J = 8.4 Hz, 1H), 7.89, 7.92(two s, 1H), 8.40, 8.45 (two br s, 1H).
I-10
   ¹H NMR(CDCl₃, δ ppm): 0.15-0.2(m, 2H), 0.55-0.6(m, 2H), 0.85-0.95 (m, 1H) , 1.15-2.0(m, 10H), 1.66(d, J = 5.7 Hz, 3H), 2.2-2.9(m, 9H), 3.14(d, J = 18 . 3 Hz, 1H) , 3.35-3.4(m, 1H) , 4.6-4.7(m, 1H) , 4.9-5.3 (br s, 1H), 5.67(s, 1H), 6.5-6.6(m, 1H), 6.63(d, J = 8.1 Hz, 1H), 7.0-7.1(m, 1H), 7.28, 7.31(two d, J = 9 Hz, 1H), 7.60, 7.66(two d, J = 8.7 Hz, 1H), 7.91, 7.92(two s, 1H), 8.35, 8.39 (two br s,1H)

Test Example 1 Effect on the retching and emesis induced by morphine
1) Test compound
   Said compound (I-1) was used.
2) Test method
   About 30 minutes after feeding, ferrets were placed into observation cages and administered with the test compound. The test compound was dissolved into 15% Solutol (registered trademark) HS15 containing 5% of N,N-dimethylacetamide and 5% of N-methylglucamine, and administered at 5 mg/kg. Thirty minutes after administration of the test compound solution, morphine at 0.6 mg/kg was subcutaneously administered in a solution volume of 1 mL/kg, and the retching and emesis symptoms were visually observed for 30 minutes after administration of morphine.
   The frequency, latency (the period from the administration of morphine to the initial occurrence of emetic symptom) and duration (the period from the initial emesis to the final emesis) of retching (rhythmical contraction of abdomen), emesis (emetic behavior to discharge the vomit or similar behavior) and both the symptoms were tabulated.
   Meanwhile, the latency of a case showing no emesis was assumed to be 30 minutes that were the longest time of observation, and a duration of less than 1 minute after the occurrence of emesis was expressed as 1 minute for the sake of convenience.
   The results are shown in Table 1.

**[Table 1]**

| | | Dose (mg/kg) | Number of individuals with the symptom (Number of individuals with the symptom/Number of cases) | Latent period (min) | Duration (min) | Occurrence times |
|---|---|---|---|---|---|---|
| Retching | Control ^{a)} | - | 3/3 | 3±1 | 9±2 | 47±13 |
| | I-1 | 0.05 | 0/6 | 30±0 | 0±0 | 0±0 |
| | | 0.5 | 0/6 | 30±0 | 0±0 | 0±0 |
| | | 5 | 0/6 | 30±0 | 0±0 | 0±0 |
| Emesis | Control ^{a)} | - | 3/8 | 4±1 | 7±3 | 4±2 |
| | I-1 | 0.05 | 0/6 | 30±0 | 0±0 | 0±0 |
| | | 0.5 | 0/6 | 30±0 | 0±0 | 0±0 |
| | | 5 | 0/6 | 30±0 | 0±0 | 0±0 |
| Retching + emesis | Control ^{a)} | - | 3/3 | 3±1 | 9±2 | 51±14 |
| | I-1 | 0.05 | 0/6 | 30±0 | 0±0 | 0±0 |
| | | 0.5 | 0/6 | 30±0 | 0±0 | 0±0 |
| | | 5 | 0/6 | 30±0 | 0±0 | 0±0 |

| | | | | | | |
|---|---|---|---|---|---|---|
| Each value expresses "Mean ± Standard error." a) = 0.25 mL/kg of 15% Solutol (registered trademark) HS15 containing 15% of N,N-dimethylacetamide and 5% of N-methylglucamine | | | | | | |

From the above results, it is indicated that the compound (I) exhibits antagonism against the retching and emesis induced by the administration of morphine.

Test Example 2 Test on the ability of penetration to the brain
1) test compound
   The mesylate of the compound (I-1) was used.
2) Animal used: Crj:CD(SD)IGS strain male rats, 7 weeks old
3) Dosing solution
   One mg of the test compound was dissolved into 600 microliters of N,N-dimethylacetamide (DMAA), and 1.4 mL of propylene glycol (PG) was added. The mixture was sufficiently stirred.
4) Samples: Blood and brain*
5) Experimental method
   Non-fasted rats (n = 2) were intravenously administered with 0.5 mg/mL/kg of the test compound, and 30 minutes after administration, blood was drawn from their jugular veins. The concentrations of the parent compound in the brain and the plasma were measured, and Kp value was calculated.
   Meanwhile, 3 days before the experiment, blood drawing tubes were inserted into the right jugular veins of the rats, and thereafter, they were housed as usual.
6) Sample processing methods
   The blood was centrifuged (3000 rpm, 10 minutes, 4°C) and all the plasma fractions were taken in a tube. The brain was homogenized as a 25% suspension. The quantitative determination was performed by LC/MS/.MS method.
7) Result
   From the obtained parent compound concentrations, the Kp value was calculated and found to be 0.03.
   Therefore, it is indicated that the compound of this invention is unlikely to pass the blood-brain barrier and can relieve the retching and emesis induced as side effect without inhibiting the analgesic action of the opioid receptor agonist.

### Test Example 3 Method for the binding assay of opioid receptor

1) Method for preparing a membrane preparation for the binding assay
   A cerebrum of rat (Slc:SD) stored at -80°C was used for the assay. The cerebrum was weighed, and ice-cooled 10 mM Tris-HCl buffer (pH 7. 0) was added to the cerebrum by an amount corresponding to 20 times the amount of the cerebrum. The mixture was homogenized (25000 rpm, 30 seconds) by Histocolon (NITI-ON) and centrifuged at 36600xg for 20 minutes. The obtained pellets were re-suspended with 15 mL of the same buffer, and the mixture was similarly treated by Histocolon and centrifuged. This washing process was performed twice. After completion of centrifugation, the obtained pellets were re-suspended with 15 mL of 50 mM Tris-HCl buffer (pH 7.4), and the mixture was treated by Histocolon and re-suspended into the same buffer to ensure that the total amount of the buffer became ten times the amount of the pellets. Thus, a crude membrane fraction (Life Sci. 48, 111-116, 1991) was obtained. The prepared membrane preparation was stored under freeze at -80°C. For testing, it was quickly thawed, centrifuged and homogenized by Histocolon as described above. And it was diluted by 50 mM Tris-HCl buffer (pH 7.4) to about 900 µg/mL, for use in the experiment. To measure the protein concentration of the membrane preparation, Micro BCA Protein Assay Kit (PIERCE) was used.
2) Method for performing the δ receptor binding assay, and data analysis
   Test compounds were serially diluted up to tenfold. Ten µL of each of the test compound solution was poured in a tube, added 10 µL of [³H]-DADLE (51.5Ci/mmol:Perkin Elmer) as a ligand at a final concentration of 3 nM. Four hundred and eighty microliters of the rat cerebrum membrane fraction containing 100 mM choline chloride, 3 mM MnCl₂ and 100 nM DAMGO was poured in a tube and incubated at 25°C for 2 hours. After completion of incubation, it was aspiration-filtered on a Whatman GF/C filter pretreated by 0.5% polyethyleneimine, and the residue was washed with 2.5 mL of ice-cooled 10 mM Tris-HCl buffer (pH 7.4) 4 times. After completion of washing, the filter was placed into a mini-vial for a liquid scintillation counter, and 5 mL of a scintillator (Cleasol I) was added. It was allowed to stand overnight, and radioactivity for 3 minutes was measured using the liquid scintillation counter Tri-Carb 2200CA (PACKARD). DMSO was used for the total bound (TB) for data analysis and 20 µM levallorphan was used for non-specific bound (NB). The Ki value of the test compound was calculated using the KD value (2.93 nM) of the ligand obtained beforehand by Scatchard plot analysis.
   The results are shown in Table 2.

**[Table 2]**

| Compound | Ki(nM) |
|---|---|
| I-1 | 0.62 |
| I-2 | 1.7 |
| 1-3 | 1.06 |
| 1-4 | 1.14 |
| 1-8 | 1.38 |
| 1-9 | 1.25 |

### Formulation Example 1

Granules containing the following ingredients are produced.

| Ingredients: | Compound represented by formula (I) | 10 mg |
|---|---|---|
| | Lactose | 700 mg |
| | Corn starch | 274 mg |
| | HPC-L | 16 mg |
| | | 1000 mg |

The compound represented by the formula (I) and lactose are passed through a 60-mesh sieve. Corn starch is passed through a 120-mesh sieve. They are mixed by a V mixer. To the mixed powder, added is HPC-L (hydroxypropyl cellulose with a low viscosity) aqueous solution, and the mixture is kneaded, granulated (extrusion granulation pore size 0.5 to 1 mm), and dried. The obtained dried granules are sieved by a vibration sieve (12/60-mesh), to obtain granules.

### Formulation Example 2

Granules to be packed in a capsule, containing the following ingredients, are produced.

| Ingredients: | Compound represented by formula (I) | 15 mg |
|---|---|---|
| | Lactose | 90 mg |
| | Corn starch | 42 mg |
| | HPC-L | 3 mg |
| | | 150 mg |

The compound represented by the formula (I) and lactose are passed through a 60-mesh sieve. Corn starch is passed through a 120-mesh sieve. They are mixed, and to the mixed power, added is HPC-L solution. The mixture is kneaded, granulated and dried. The obtained dried granules are dressed, and 150 mg of them are packed in a No. 4 hard gelatin capsule.

### Formulation Example 3

A tablet containing the following ingredients is produced.

| Ingredients: | Compound represented by formula (I) | 10 mg |
|---|---|---|
| | Lactose | 90 mg |
| | Microcrystalline cellulose | 30 mg |
| | CMC-Na | 15 mg |
| | Magnesium stearate | 5 mg |
| | | 150 mg |

The compound represented by the formula (I), lactose, microcrystalline cellulose and CMC-Na (carboxymethyl cellulose sodium salt) are passed through a 60-mesh sieve, and they are mixed. To the mixed powder, added is magnesium stearate, to obtain a mixed powder to be tableted. The mixed powder is directly tableted to obtain a 150 mg tablet.

### Formulation Example 4

The following ingredients are heated, mixed and sterilized to prepare an injection.

| Ingredients: | Compound represented by formula (I) | 3 mg |
|---|---|---|
| | Nonionic surfactant | 15 mg |
| | Purified water for injection | 1 mg |

### INDUSTRIAL APPLICABILITY

The compounds of this invention are useful as remedies and/or preventives for retching and/or emesis, and also as agents for relieving and/or preventing the side effect of an opioid receptor agonist.

## Claims

1. A compound represented by the following formula (I): 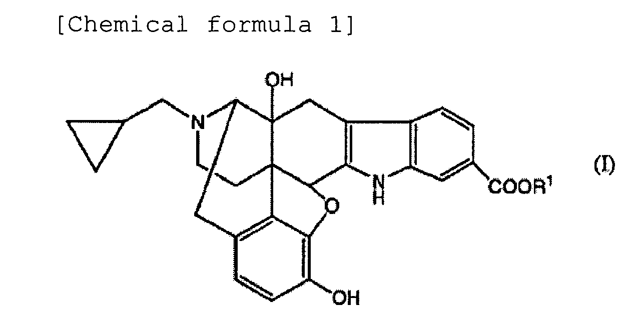 {where R¹ denotes hydrogen or -CHR^{A}R^{B} (where R^{A} denotes a lower alkoxycarbonyloxy, cycloalkoxycarbonyloxy, acyloxy or a group represented by 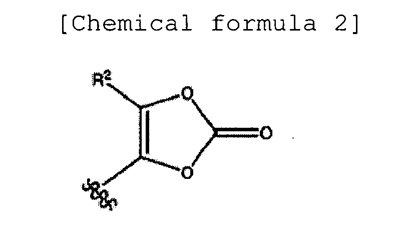 (where R² denotes hydrogen or a lower alkyl with or without a substituent), and R^{B} denotes hydrogen or methyl}, or any of pharmaceutically acceptable salts thereof or any of solvates thereof.

2. A compound or any of pharmaceutically acceptable salts thereof or any of solvates thereof, according to claim 1, wherein R¹ denotes hydrogen.

3. A drug composition comprising the compound or any of pharmaceutically acceptable salts thereof or any of solvates thereof as set forth in claim 1.

4. An opioid receptor antagonist comprising the compound or any of pharmaceutically acceptable salts thereof or any of solvates thereof as set forth in claim 1.

5. A drug composition, according to claim 3, wherein the compound or any of pharmaceutically acceptable salts thereof or any of solvates thereof as set forth in claim 2 is a medicinal active ingredient.

6. A drug composition, according to claim 3, which is a remedy and/or preventive for retching and/or emesis.

7. A drug composition, according to claim 3, which is an agent for reliving and/or preventing the side effect induced by a compound having opioid receptor agonism.

8. A remedy and/or preventive, according to claim 7, wherein the side effect is retching and/or emesis.

9. A remedy and/or preventive, according to claim 7 or 8, wherein the compound having opioid receptor agonism is morphine, oxycodone, any of pharmaceutically acceptable salts thereof or any of solvates thereof.

10. Use of the compound or any of pharmaceutically acceptable salts thereof or any of solvates thereof as set forth in claim 1 or 2, for producing a drug having opioid receptor antagonism.

11. Use of the compound or any of pharmaceutically acceptable salts thereof or any of solvates thereof as set forth in claim 1 or 2, for producing a drug for curing and/or preventing retching and/or emesis.

12. Use of the compound or any of pharmaceutically acceptable salts thereof or any of solvates thereof as set forth in claim 1 or 2, for producing a drug for relieving and/or preventing the side effect induced by a compound having opioid receptor agonism.

13. A method for inhibiting an opioid receptor, **characterized by** administering the compound or any of pharmaceutically acceptable salts thereof or any of solvates thereof as set forth in claim 1 or 2.

14. A method for curing and/or preventing retching and/or emesis, **characterized by** administering the compound or any of pharmaceutically acceptable salts thereof or any of solvates thereof as set forth in claim 1 or 2.

15. A method for relieving and/or preventing the side effect induced by a compound having opioid receptor agonism, **characterized by** administering the compound or any of pharmaceutically acceptable salts thereof or any of solvates thereof as set forth in claim 1 or 2.

16. An analgesic comprising a compound having opioid receptor agonism and the compound or any of pharmaceutically acceptable salts thereof or any of solvates thereof as set forth in claim 1 or 2 in an amount effective for relieving and/or preventing the side effect induced by the administration of said compound, in combination.

17. An analgesic comprising a compound having opioid receptor agonism and the compound or any of pharmaceutically acceptable salts thereof or any of solvates thereof as set forth in claim 1 or 2 in an amount effective for curing and/or preventing the retching and/or emesis induced by the administration of said compound, in combination.
